# EUROPÄISCHE PATENTSCHRIFT

(11) **EP 3 171 934 B1**
(45) Veröffentlichungstag und Bekanntmachung des Hinweises auf die Patenterteilung: **17.10.2018**
(21) Anmeldenummer: 15731849.4
(22) Anmeldetag: 25.06.2015
(51) Int. Cl.: A61N 1/36

(54) **VORRICHTUNG ZUR AUFBRINGUNG EINES TRANSKUTANEN ELEKTRISCHEN STIMULATIONSREIZES**
DEVICE FOR APPLYING A TRANSCUTANEOUS ELECTRICAL STIMULATION SIGNAL
DISPOSITIF D'APPLICATION D'UN STIMULUS D'EXCITATION ÉLECTRIQUE TRANSCUTANÉE

(30) Priorität: 27.07.2014 DE 102014010882
(43) Veröffentlichungstag der Anmeldung: 31.05.2017
(73) Patentinhaber: cerbomed GmbH, 91052 Erlangen (DE)
(72) Erfinder: ZSCHAECK, Thomas, 90427 Nürnberg (DE); FRENKEL, Wolf Gerhard, 72514 Inzigkofen-Engelswies (DE); HYCA, Martin, 85221 Dachau (DE); HARTLEP, Andreas, 83607 Holzkirchen (DE)
(74) Vertreter: Gosdin, Michael
(86) Internationale Anmeldenummer: PCT/EP2015/001279
(87) Internationale Veröffentlichungsnummer: WO 2016/015802

(56) Entgegenhaltungen:
- WO-A1-92/08516
- WO-A2-2004/000413
- WO-A2-2010/032112
- WO-A2-2012/082960
- CA-A1- 2 707 351
- CN-A- 101 678 206
- US-A1- 2007 150 027

## Beschreibung

Die Erfindung betrifft eine Vorrichtung zur Aufbringung eines transkutanen elektrischen Stimulationsreizes auf die Oberfläche eines Abschnittes des menschlichen Ohrs, die eine Anzahl Elektroden aufweist, wobei die Vorrichtung eine Steuerungseinrichtung umfasst, die zur Einleitung eines über die Elektroden fließenden Stimulationsstroms gemäß einem vorgegebenen Verlauf des Stimulationsstroms über der Zeit ausgebildet ist.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Vorrichtung der eingangs genannten Art ist aus der DE 10 2010 054 165 B3 bekannt. Hier ist eine Vorrichtung zur transkutanen Stimulation des Vagusnervs des menschlichen Körpers beschrieben, deren Elektrodenkopf mit zwei Elektroden im Bereich der Cymba conchae angeordnet wird; eine solche Positionierung der Elektroden hat sich als vorteilhaft erwiesen. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae.

Dabei ist es typisch, periodische Stromsequenzen über die Elektroden auf den Vagusnerv aufzugeben, wobei mit relativ hohen Frequenzen von mindestens 25 Hz, teilweise auch mit erheblich höheren Frequenzen gearbeitet wird.

Zum diesbezüglichen Stand der Technik wird auf die US 2005/0165460 A1, auf die DE 10 2010 022 026 A1, auf die DE 10 2011 009 528 A1 und auf die US 2006/0064139 A1 hingewiesen. Weitere Lösungen zeigen die WO 2004/000413 A2, die WO 2012/082960 A2, die WO 2010/032112 A2, die CA 2 707 351 A1, die WO 92/08516 A1, die US 2007/0150027 A1 und die CN 101 678 206 A.

Es hat sich dabei herausgestellt, dass für manche Krankheitsbilder hiermit kein zufriedenstellender Erfolg erreichbar ist. Hierbei ist insbesondere an die Behandlung von Migräne gedacht, die mit herkömmlichen Stimulationsvorrichtungen manchmal nicht effektiv behandelbar ist.

Der vorliegenden Erfindung liegt daher die **Aufgabe** zugrunde, eine Vorrichtung der eingangs genannten Art bereitzustellen, mit der es möglich wird, eine effektive Behandlung bestimmter neurologischer Krankheitsbilder per transkutaner Vagusnervstimulation zu ermöglichen.

Die **Lösung** dieser Aufgabe durch die Erfindung ist dadurch gekennzeichnet, dass die Stimulationseinrichtung in der Stromstärke periodisch wiederkehrende Ströme erzeugt, wobei die Frequenz der wiederkehrenden Ströme zwischen 0,75 Hz und 1,25 Hz liegt, wobei die periodisch wiederkehrenden Ströme biphasisch sind, d. h. der Strom weist positive und negative Teile auf, wobei die Amplitude des Stimulationsstroms zwischen 0,02 mA und 8,0 mA liegt, wobei die Stimulationseinrichtung Stromimpulse einer vorgegebenen zeitlichen Dauer erzeugt, die durch stromfreie Phasen getrennt sind, wobei die Dauer der Stromimpulse zwischen 100 µs und 1.000 µs liegt und wobei der Funktionsverlauf der Stromimpulse trapezförmig ist.

Die Dauer der Stromimpulse liegt dabei bevorzugt zwischen 200 µs und 300µs.

Die Amplitude des Stimulationsstroms liegt bevorzugt zwischen 0,02 mA und 5,0 mA.

Die Maximalwerte des Stroms der Stromimpulse können zumindest über eine vorgegebene Zeit ansteigend sein. Demgemäß steigen die einzelnen Stromimpulse rampenförmig an.

Eine weitere Fortbildung sieht vor, dass die Stimulationseinrichtung nach Erzeugung einer Anzahl Stromimpulse einer vorgegebenen zeitlichen Dauer über eine sich anschließende vorgegebene Pausenzeit keinen Strom auf die Elektroden aufgibt. Hierbei ist bevorzugt vorgesehen, dass die Zeit, in der die Anzahl Stromimpulse erzeugt wird, zu der Zeit, in der kein Strom auf die Elektroden aufgegeben wird, in einem Verhältnis zwischen 1 : 1 bis 1 : 100 steht. Somit kann also vorgesehen werden, dass nach einer Anzahl von Stromstößen der genannten Art und mit der erwähnten Frequenz eine stimulationsfreie Pausenzeit eingehalten wird. Dabei kann beispielsweise eine Stimulation über 5 Sekunden erfolgen, woraufhin eine stimulationsfreie Pausenzeit von mehreren Minuten erfolgt (bei einer Pausenzeit von 8 Minuten läge das genannte Verhältnis dann beispielsweise bei ca. 1 : 100). Die Auslösung weiterer Stromstöße kann natürlich beispielsweise auch direkt durch den Patienten erfolgen, der zu gegebener Zeit Einzel-Bursts (s. unten) veranlasst.

Demgemäß kann zusammengefasst gesagt werden, dass sich folgender transkutaner Stimulationsstrom als besonders vorteilhaft erwiesen hat, um insbesondere eine Therapie bei Migräne zu ermöglichen:
Der Pulsstrom (Amplitude des Stimulationsstroms) liegt bevorzugt zwischen 0,02 und 8,0 mA, wobei ein biphasischer Impuls eingesetzt wird. Die Pulsbreite liegt bevorzugt zwischen 100 µs und 1.000 µs (bevorzugt bei 250 µs), die Frequenz zwischen 0,01 Hz und 10 Hz (bevorzugt bei 1 Hz).

Eingesetzt werden auch vorzugsweise Burst-Zyklen (also Bündel von einzelnen Stromstößen) mit intermittierender Stimulation; die Burst-Zyklen können dabei intermittierend mit an- und abschwellender Stimulation erfolgen.

Es hat sich herausgestellt, dass bestimmte neurologische Erkrankungen besonders gut auf transkutan aurikulär applizierte Stimulationsströme im eher niedrigen Frequenzbereich ansprechen. Hier sei vor allem Migräne als Krankheitsbild genannt.

Insofern betrifft die Erfindung auch ein Verfahren zur Behandlung von Migräne mit der genannten Ausbildung des transkutanen Stimulationsstroms.

In der Zeichnung ist ein Ausführungsbeispiel der Erfindung dargestellt. Es zeigen:
- Fig. 1: schematisch den Verlauf des Stimulationsstroms über der Zeit,
- Fig. 2: einen vergrößerten Ausschnitt aus dem Verlauf gemäß Fig. 1,
- Fig. 3: eine Aufstellung primärer und sekundärer Behandlungsergebnisse bei Migräne und
- Fig. 4: eine Ergebnisübersicht über die Wirkung der erfindungsgemäßen Behandlung bei Migräne.

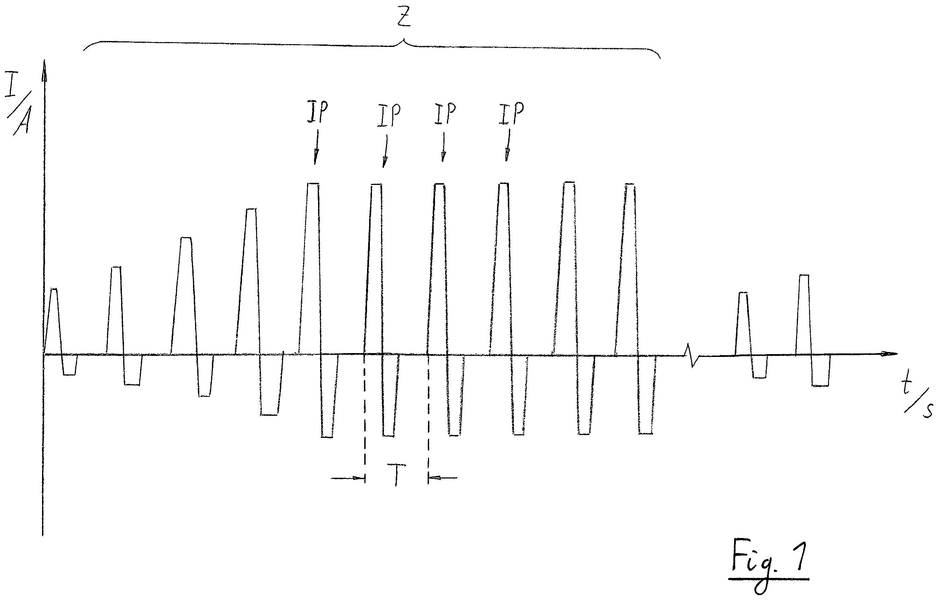

In Fig. 1 ist dargestellt, wie der Verlauf des Stimulationsstroms I über der Zeit t aussehen kann. Zu erkennen ist zunächst ein Zyklus Z, wobei während des Zyklus Z eine Anzahl von Stromimpulsen IP von der Steuerung der Stimulationsvorrichtung ausgelöst wird. Die Stimulationsvorrichtung muss hier nicht weiter erwähnt werden, da sie als solche hinlänglich bekannt ist. Zu den vorbekannten Vorrichtungen, die im Zusammenhang mit dem vorliegenden Konzept eingesetzt werden können, sei ausdrücklich auf die DE 10 2010 054 165 B3 der Patentinhaberin Bezug genommen wird, wo sich nähere Erläuterungen finden. Die Vorrichtung ist demnach ausgebildet, um im Bereich des Vagusnervs am Ohr der die Vorrichtung benutzenden Person angebracht zu werden. Damit kann eine transkutane Stimulation des Vagusnervs vorgenommen werden.

In Fig. 1 ist zu erkennen, dass nach dem Zyklus Z eine Pausenzeit eingehalten wird, an den sich ein weiterer Zyklus Z anschließt (bzw. weitere Zyklen Z), von dem in Fig. 1 allerdings nur die ersten beiden Stromimpulse dargestellt sind. Der Stimulationsstrom I hat gemäß dem Ausführungsbeispiel einen trapezförmigen Verlauf über der Zeit t. Dabei sind die sich periodisch wiederholenden einzelnen Stromimpulse IP eine Periodendauer T voneinander beabstandet, so dass sich für die Anregung eine Frequenz f = 1/T ergibt, die gemäß dem Kern der vorgeschlagenen Vorgehensweise bevorzugt (mit der genannten Bandbreite) bei ca. 1 Hz liegt.

Dem steht nicht entgegen, dass die Amplituden des Stimulationsstroms I über die einzelnen Stromimpulse IP nicht zwingend gleich bleiben. Es kann - wie in Fig. 1 dargestellt - ein ansteigender Verlauf, d. h. ein "ramp-up", zu Beginn des Zyklus Z erfolgen.

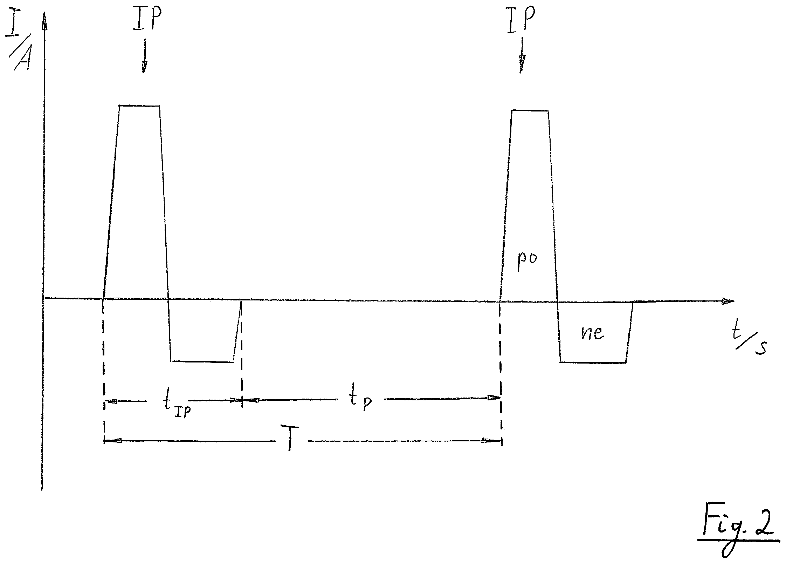

Für zwei aufeinander folgende Stromimpulse IP, die eine gleiche Höhe des Stimulationsstroms I vorsehen, ist in Fig. 2 eine vergrößerte Darstellung skizziert. Zu sehen ist, dass die biphasisch ausgebildete Stromform (d. h. neben einem positiven Bereich po ist ein negativer Bereich ne vorgesehen) trapezförmig ausgestaltet ist, wobei der Stimulationsstrom I über eine Dauer t_{IP} des Stromimpulses fließt. Hieran schließt sich eine stromfreie Phase an, die eine Dauer von t_{P} hat. Die gesamte Pulsbreite des Stromimpulses IP beträgt also t_{IP}. Hieraus lässt sich unter Betrachtung zweier aufeinander folgender Stromimpulse IP die Periodendauer T und hieraus wiederum die Frequenz f = 1/T bestimmen, die generell zwischen 0,01 Hz und 10 Hz liegen soll, besonders bevorzugt indes bei ca. 1 Hz.

Da die bevorzugte Dauer t_{IP} eines Stromimpulses bei nur ca. 250 µs liegt, ist ersichtlich, dass die Darstellung in den Figuren keinesfalls maßstäblich ist.

Während ein trapezförmiger oder rechteckförmiger Stromverlauf bevorzugt ist, kann dieser auch anders gewählt werden. Dies kann bis hin zu einem sinusförmigen Verlauf des Stimulationsstroms erfolgen, der dann mit der genannten Frequenz über die Elektroden aufgegeben wird.

Während also im Stand der Technik bislang mit Anregungsfrequenzen gearbeitet wird, die bei der transkutanen Stimulation des Vagusnervs zumeist in der Größenordnung von Millisekunden liegt (beispielsweise bei einer Pulsperiode von 40 ms, entsprechend einer Frequenz von 25 Hz), sieht die vorliegende Idee vor, dass hier größenordnungsmäßig andere Bereiche zum Einsatz kommen, nämlich Pulsperioden bevorzugt von 1.000 ms, entsprechend einer Frequenz von 1 Hz.

Der genannte Wertebereich hat vorliegenden Studien zufolge eine hohe Wirksamkeit in der prophylaktischen Behandlung chronischer Migräne gezeigt.

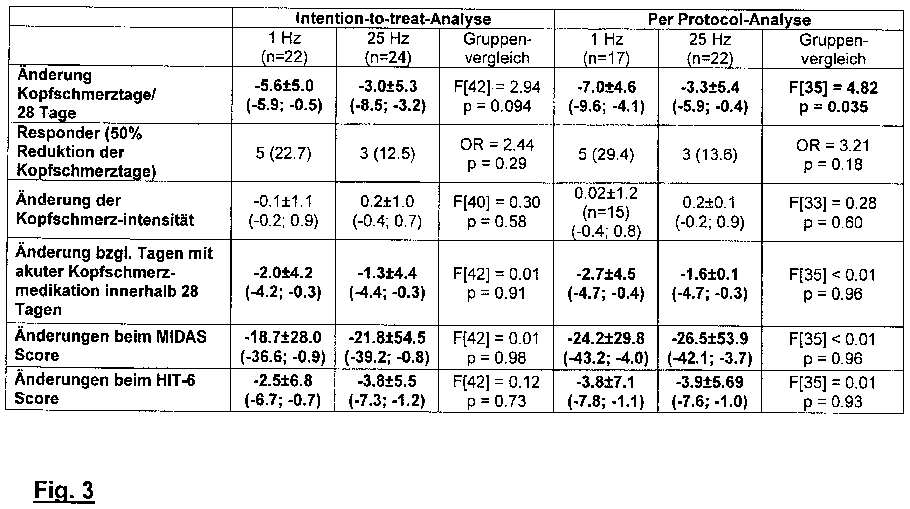

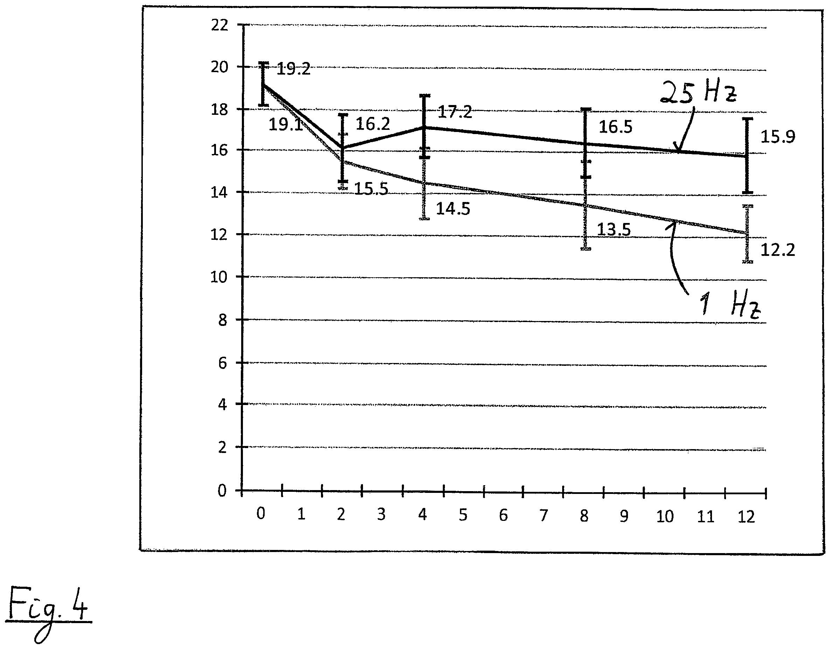

Dies ergibt sich insbesondere aus den Darstellungen, die in den Figuren 3 und 4 dargestellt sind.

Es wurde versucht festzustellen, mit welchen transkutanen Stimulationsreizen das Krankheitsbild der Migräne besonders wirkungsvoll behandelt werden kann. Hierzu hat die Anmelderin umfassende Studien durchgeführt.

Das Ziel der Studie war der Nachweis von Wirksamkeit und Sicherheit der transkutanen Stimulation des aurikulären Vagusnervs (t-VNS) bei der Behandlung der chronischen Migräne.

Hierzu wurde eine monozentrische, randomisierte, kontrollierte, doppelblinde Studie durchgeführt. Nach einem Monat Vorbeobachtung wurden die an chronischer Migräne leidenden Patienten randomisiert zugeteilt, entweder zu einer 25 Hz- oder 1 Hz-Stimulation des sensorischen vagalen Hautareals (Cymba Conchae) am linken Ohr. Mit einem batteriebetriebenen Handgerät wurde über 4 Stunden täglich während 3 Monaten stimuliert. Verglichen wurden die Kopfschmerztage per 28 Tage zwischen dem Ausgangsbefund und dem letzten Behandlungsmonat. Mithilfe eines Fragebogen "Headache Impact Test (HIT-6)" und "Migraine Disability Assessment (MIDAS)" wurde zusätzlich die durch Kopfschmerzen bedingte Behinderung der Patienten im täglichen Leben bestimmt.

Das Ergebnis ist in Figur 3 in Form der dort dargestellten Tabelle zu sehen: Von den 46 randomisierten Patienten beendeten 40 die Studie ("per protocol"). In der "per protocol"-Analyse erfuhren die Patienten der 1 Hz-Gruppe eine signifikant größere Reduktion der Kopfschmerztage per 28 Tage als die Patienten in der 25 Hz-Gruppe (-7.0 ± 4.6 vs. -3.3 ± 5.4 Tage, p = 0.035). 29,4 % der Patienten in der 1 Hz-Gruppe profitierten von einer Reduktion der Kopfschmerztage ≥50%, verglichen mit nur 13,3 % bei der 25 Hz-Gruppe. Die Werte für die Fragebogen HIT-6 und MIDAS verbesserten sich ebenfalls signifikant, und zwar in beiden Gruppen, ohne Gruppendifferenz.

Es wurden keine auf die Behandlung zurückzuführenden schwerwiegenden unerwünschten Ereignisse registriert.

Hieraus ergibt sich die Schlussfolgerung, dass die Behandlung der chronischen Migräne durch t-VNS mit 1 Hz sicher und effektiv ist. Die mittlere Reduktion von Kopfschmerztagen nach 12 Wochen Behandlung war höher als diejenige, die in der Literatur für andere Neurostimulationsmethoden berichtet wird.

In Figur 3 sind Änderungen angegeben zwischen der 4-wöchigen Vorbeobachtungszeit und den letzten 4 Wochen der 12-wöchigen Behandlungsperiode. Eingetragen sind Mittelwerte, Standardabweichung und das 95 %-Konfidenzintervall. Bei der Analyse der Responder sind die Anzahl der Patienten und der Prozentsatz von der jeweiligen Gruppe vermerkt. Signifikante Differenzen sind fett gedruckt. Die Anzahl der Patienten ist in Klammern angegeben, sofern unterschiedlich von der Gesamtgruppe.

Primäre Zielvariablen: Änderung bei den Kopfschmerztagen pro 28 Tage, MIDAS (Migraine Disability Assessment) und HIT (Headache Impact Test).

Dieses Ergebnis ist noch einmal in Figur 4 illustriert: Hier ist auf der Abszisse des dargestellten Graphen die Behandlungszeit in Wochen dargestellt und auf der Ordinate die Kopfschmerztage pro Monat.

Klar zu erkennen ist, dass bei der erfindungsgemäßen Stimulation mit 1 Hz (unterer Kurvenverlauf) im Vergleich mit derjenigen mit 25 Hz (oberer Kurvenverlauf) eine signifikante Verbesserung über der Behandlungszeit auftritt. Während zu Beginn der Behandlung die Anzahl der Kopfschmerztage im Falle chronischer Migräne bei 19,1 Tagen bzw. 19,2 Tagen liegt, verändert sich dies mit zunehmender Zeit zusehends: Nach 12 Behandlungswochen sinkt die Anzahl der Kopfschmerztage bei der Behandlung mit 25 Hz auf 15,9, während die Anzahl der Kopfschmerztage bei der Behandlung mit 1 Hz wesentlich stärker auf 12,2 abfällt.

Demgemäß ist mit der anspruchsgemäßen Stimulation in überraschender Weise eine signifikante Verbesserung der Behandlung chronischer Migräne mittels transkutaner Stimulation des Vagusnervs möglich, da die Stimulation mit dieser Frequenz ursprünglich nur als unwirksame Scheinstimulation intendiert war.

### Bezugszeichenliste:

- IP: Stromimpuls

- I: Stimulationsstrom
- t: Zeit
- f: Frequenz (s⁻¹)
- T: Periodendauer (s)

- t_{IP}: Dauer des Stromimpulses
- t_{P}: Dauer der stromfreien Phase

## Patentansprüche

1. Vorrichtung zur Aufbringung eines transkutanen elektrischen Stimulationsreizes auf die Oberfläche eines Abschnittes des menschlichen Ohrs, die eine Anzahl Elektroden aufweist, wobei die Vorrichtung eine Steuerungseinrichtung umfasst, die zur Einleitung eines über die Elektroden fließenden Stimulationsstroms (I) gemäß einem vorgegebenen Verlauf des Stimulationsstroms (I) über der Zeit (t) ausgebildet ist,
wobei die Stimulationseinrichtung in der Stromstärke (I) periodisch wiederkehrende Ströme erzeugt, wobei die Frequenz (f = 1/T) der wiederkehrenden Ströme zwischen 0,75 Hz und 1,25 Hz liegt,
wobei die periodisch wiederkehrenden Ströme biphasisch sind,
wobei die Amplitude des Stimulationsstroms (I) zwischen 0,02 mA und 8,0 mA liegt,
wobei die Stimulationseinrichtung Stromimpulse (IP) einer vorgegebenen zeitlichen Dauer (t_{IP}) erzeugt, die durch stromfreie Phasen (t_{P}) getrennt sind, wobei die Dauer (t_{IP}) der Stromimpulse (IP) zwischen 100 µs und 1.000 µs liegt und
wobei der Funktionsverlauf der Stromimpulse (IP) trapezförmig ist.

2. Vorrichtung nach Anspruch 1, **dadurch gekennzeichnet, dass** die Dauer (t_{IP}) der Stromimpulse (IP) zwischen 200 µs und 300 µs liegt.

3. Vorrichtung nach Anspruch 1 oder 2, **dadurch gekennzeichnet, dass** die Amplitude des Stimulationsstroms (I) zwischen 0,02 mA und 5,0 mA liegt.

4. Vorrichtung nach einem der Ansprüche 1 bis 3, **dadurch gekennzeichnet, dass** die Maximalwerte des Stroms (I) der Stromimpulse (IP) zumindest über eine vorgegebenen Zeit ansteigend sind.

5. Vorrichtung nach einem der Ansprüche 1 bis 4, **dadurch gekennzeichnet, dass** die Stimulationseinrichtung nach Erzeugung einer Anzahl Stromimpulse (IP) einer vorgegebenen zeitlichen Dauer über eine sich anschließende vorgegebene Pausenzeit keinen Strom auf die Elektroden aufgibt.

6. Vorrichtung nach Anspruch 5, **dadurch gekennzeichnet, dass** die Zeit, in der die Anzahl Stromimpulse (IP) erzeugt wird, zu der Zeit, in der kein Strom auf die Elektroden aufgegeben wird, in einem Verhältnis zwischen 1 : 1 bis 1 : 100 steht.

## Claims

1. Device for the application of a transcutaneous electric stimulation stimulus onto the surface of a section of the human ear, which comprises a number of electrodes, wherein the device comprises a controlling device which is designed for the application of a stimulation current (I) which is applied via the electrodes according to a predetermined run of the stimulation current (I) along the time (t),
wherein the stimulation device creates a periodic recurrent stimulation current (I), wherein the frequency (f = 1/T) of the recurrent stimulation current is between 0.75 Hz and 1.25 Hz,
wherein the recurrent stimulation currents are biphasic,
wherein the amplitude of the stimulation current (I) is between 0.02 mA and 8.0 mA,
wherein the stimulation device creates current pulses (IP) with a predetermined duration (t_{IP}) which are interrupted by current-free phases (t_{P}), wherein the duration (t_{IP}) of the current pulses (IP) is between 100 µs and 1,000 µs and
wherein the course of the function of the current pulses (IP) is trapezoid.

2. Device according to claim 1, **characterized in that** the duration (t_{IP}) of the current pulses (IP) is between 200 µs and 300 µs.

3. Device according to claim 1 or 2, **characterized in that** the amplitude of the stimulation current (I) is between 0.02 mA and 5.0 mA.

4. Device according to one of claims 1 to 3, **characterized in that** the maximum values of the stimulation current (I) of the current pulses (IP) are increasing at least along a predetermined time.

5. Device according to one of claims 1 to 4, **characterized in that** the stimulation device does not apply a current onto the electrodes after the creation of a number of current pulses (IP) of a predetermined duration during a following pause time.

6. Device according to claim 5, **characterized in that** the time, in which the number of current pulses (IP) is created, and the time, in which no current is applied onto the electrodes, are in a relationship between 1 : 1 and 1 : 100.

## Revendications

1. Arrangement pour appliquer un stimulus électrique transcutané sur la surface d'une portion de l'oreille humaine, laquelle possède une pluralité d'électrodes, l'arrangement comportant un dispositif de commande qui est configuré pour introduire un courant de stimulation (I) s'écoulant par le biais des électrodes conformément à une courbe prédéfinie du courant de stimulation (I) en fonction du temps (t),
le dispositif de stimulation générant des courants dont l'intensité de courant (I) se répète périodiquement, la fréquence (f = 1/T) des courants périodiques étant comprise entre 0,75 Hz et 1,25 Hz,
les courants qui se répètent périodiquement étant biphasés,
l'amplitude du courant de stimulation (I) étant comprise entre 0,02 mA et 8,0 mA,
le dispositif de stimulation générant des impulsions de courant (IP) ayant une durée temporelle (t_{IP}) prédéfinie, lesquelles sont séparées par des phases sans courant (t_{P}), la durée (t_{IP}) des impulsions de courant (IP) étant comprise entre 100 µs et 1 000 µs et la courbe de la fonction des impulsions de courant (IP) étant trapézoïdale.

2. Arrangement selon la revendication 1, **caractérisé en ce que** la durée (t_{IP}) des impulsions de courant (IP) est comprise entre 200 µs et 300 µs.

3. Arrangement selon la revendication 1 ou 2, **caractérisé en ce que** l'amplitude du courant de stimulation (I) est comprise entre 0,02 mA et 5,0 mA.

4. Arrangement selon l'une des revendications 1 à 3, **caractérisé en ce que** les valeurs maximales du courant (I) des impulsions de courant (IP) est croissante au moins sur une durée prédéfinie.

5. Arrangement selon l'une des revendications 1 à 4, **caractérisé en ce que** le dispositif de stimulation, après avoir généré un nombre donné d'impulsions de courant (IP) d'une durée dans le temps prédéfinie, ne délivre aucun courant aux électrodes pendant un temps de pause prédéfini rattaché.

6. Arrangement selon la revendication 5, **caractérisé en ce que** le rapport entre le temps pendant lequel le nombre donné d'impulsions de courant (IP) sont générées et le temps pendant lequel aucun courant n'est délivré aux électrodes est compris entre 1:1 et 1:100.
